Europäisches Patentamt

European Patent Office

Office européen des brevets

(19)

(11) Veröffentlichungsnummer: **0 316 748 B1**

# EUROPÄISCHE PATENTSCHRIFT

(12)

(45) Veröffentlichungstag der Patentschrift: **29.07.92**

(21) Anmeldenummer: **88118673.8**

(22) Anmeldetag: **10.11.88**

(51) Int. Cl.⁵: **C12P 21/02**, //C12P21/06, C12N9/52,C07K13/00, C12N15/00

(54) **Verfahren zur selektiven Spaltung von Fusionsproteinen.**

(30) Priorität: **20.11.87 DE 3739347**

(43) Veröffentlichungstag der Anmeldung:
**24.05.89 Patentblatt 89/21**

(45) Bekanntmachung des Hinweises auf die
Patenterteilung:
**29.07.92 Patentblatt 92/31**

(84) Benannte Vertragsstaaten:
**AT BE CH DE ES FR GB GR IT LI LU NL SE**

(56) Entgegenhaltungen:
**EP-A- 0 207 402**

(73) Patentinhaber: **HOECHST AKTIENGESELL-SCHAFT**
**Postfach 80 03 20**
**W-6230 Frankturt am Main 80(DE)**

(72) Erfinder: **Dörschug, Michael, Dr.**
**Sonnenleite 20**
**W-4630 Bochum(DE)**
Erfinder: **Seipke, Gerhard, Dr.**
**Brunnenweg 4**
**W-6200 Wiesbaden(DE)**

**Beschreibung**

Die Erfindung betrifft ein Verfahren zur Herstellung von Polypeptiden oder Proteinen durch enzymatische Spaltung eines Fusionsproteins.

Die zunehmende Bedeutung der rekombinanten DNA-Technologie zur Gewinnung von Polypeptiden und Proteinen erfordert die Entwicklung neuer Verfahren für die Anreicherung und Reinigung der Produkte, die den veränderten Ausgangsmaterialien angepaßt sind.

Gegenwärtig wird eine große Anzahl von Proteinen im Mikroorganismus als Fusionsprotein synthetisiert, d.h. vor die Aminosäuresequenz des gewünschten Polypeptids setzt man die Sequenz eines Fremdproteins (F.A.O. Harston, Biochem. J. 240 (1986) 1-12). Die Fusionsproteine fallen im allgemeinen in der Zelle aufgrund ihrer Schwer- bzw. Unlöslichkeit als sogenannte Einschlußkörperchen aus und sind so vor proteolytischem Abbau geschützt. Hohe Ausbeuten und leichte Isolierbarkeit dieses primären Genproduktes sind so gewährleistet.

Um das gewünschte Polypeptid zu gewinnen, muß es aus dem Fusionsprotein jedoch durch enzymatische oder chemische Spaltung abgetrennt werden. Häufig wird mit chemischen Methoden gespalten, da diese sich am einfachsten dem schwerlöslichen Charakter des Fusionsproteins anpassen lassen. Unvollständige Spaltung oder Bildung von Nebenprodukten durch irreversible Derivatisierung von Aminosäureseitenketten werden aber praktisch bei allen chemischen Methoden beobachtet. Auch besteht stets die Gefahr unspezifischen Abbaus von Polypeptidketten (K.-K. Han et al., Int. J. Biochem. 15, (1983) 875-884). Die Anwendung der chemischen Verfahren ist zudem begrenzt, da die Spezifität der Spaltung überwiegend durch eine einzelne Aminosäure bestimmt wird, die oft natürlich auch im gewünschten Polypeptid vorhanden ist.

Enzymatische Fragmentierungen können unter wesentlich schonenderen Bedingungen durchgeführt werden. Generelle Schwierigkeiten treten hier jedoch dadurch auf, daß das schwerlösliche Fusionsprotein mit Detergentien, Harnstoff oder Guanidinhydrochlorid in Lösung gebracht und gehalten werden muß, also Bedingungen, unter denen Enzyme oft inaktiviert werden. Proteasen, die nur eine einzige spezifische Aminosäure erkennen, können häufig nicht verwendet werden, weil diese Aminosäure auch im gewünschten Protein vorhanden ist. Andererseits ist die Verfügbarkeit von Proteasen, die nur ganz bestimmte, seltene Sequenzen aus mehreren Aminosäuren erkennen und spalten, gering. Für jedes Produkt muß also ein eigenes Spaltungsverfahren gesucht werden. Wünschenswert ist also weiterhin eine universell anwendbare Spaltungsmethode, die nur an ganz bestimmten, seltenen Aminosäuresequenzen spaltet, ohne das Protein zu schädigen und die auch auf schwerlösliche Fusionsproteine anwendbar ist.

Aus der Literatur ist Lysostaphin als zellwandabbauendes Enzym bekannt, das von Staphylococcus simulans (NRRL B-2628; Sloan et al., Int. J. of Systematic Bacteriology, Vol. 32, No. 2 (1982) 170-174) ins Medium sekretiert wird. Dieses Enzym lysiert praktisch alle bekannten Staphylococcenarten, aber keine anderen Bakterienspezies. Bisher wurde angenommen, daß Lysostaphin-Endoprotease nur die Polyglycinbrücken im Mureinsacculus der Staphylococcen (Iversen und Grov, Eur. J. Biochem. 38 (1973) 293-300) sehr selektiv spaltet. Daneben sind Transpeptidierungsversuche unter Lysostaphin Katalyse mit kurzen synthetischen Glycylpeptiden bekannt (G. L. Sloan et al., Biochem. J. 167 (1977) 293-296). Überraschenderweise fanden wir nun, daß Lysostaphin-Endoprotease auch Fusionsproteine mit einer Oligo- bzw. Polyglycinsequenz spaltet. Die Einbindung dieser Sequenz in die spezifischen sterischen Verhältnisse einer bakteriellen Zellwand ist für die Selektivität der Spaltung offenbar nicht erforderlich.

Dieses ermöglicht die schonende gezielte Abspaltung des gewünschten Proteins aus einem Fusionsprotein.

Gegenstand der vorliegenden Erfindung ist ein Verfahren zur Herstellung von Polypeptiden oder Proteinen durch enzymatische Spaltung, dadurch gekennzeichnet, daß ein Fusionsprotein mit der Sequenz

$$(Y_1 \ldots Y_m) - (Gly)_{p+q} - (X_1 \ldots X_n)$$

wobei $(Y_1 \ldots Y_m)$-$(Gly)_p$ die abzuspaltende Sequenz und $(Gly)_q$-$(X_1 \ldots X_n)$ das Polypeptid oder Protein, darstellen,

| | |
|---|---|
| X und Y | unabhängig voneinander natürliche Aminosäuren, |
| m und n | Zahlen größer als 1, |
| p bzw. q | eine Zahl größer als 0 und |
| p + q | zusammen eine natürliche Zahl zwischen 2 und 100 bedeuten, falls $Y_m$ für Gly steht, kann p + q auch < 2 und p = 0 sein und falls $X_1$ für Gly steht, kann p + q auch < 2 und q = 0 sein, |

mit einer für Oligo- bzw. Polyglycinsequenzen spezifischen Endoprotease gespalten wird und das dabei freigesetzte Polypeptid oder Protein gegebenenfalls einer weiteren chemischen oder enzymatischen Behandlung unterzogen oder direkt weiterverarbeitet wird.

In Abhängigkeit von der Größe der Indices p bzw. q unterliegt die abzuspaltende Sequenz bzw. das Polypeptid oder Protein gegebenenfalls weiteren enzymatischen Spaltungen.

$(Y_1 ... Y_m)$ ist eine natürliche oder artifizielle Proteinsequenz, wie sie üblicherweise zur Herstellung von Fusionsproteinen herangezogen wird. In Frage kommen beispielsweise $\beta$-Galactosidase, Enzyme des Tryptophan-Stoffwechsels oder Teile dieser Proteinmoleküle, die im allgemeinen zu unlöslichen Produkten führen, aber auch Polypeptidsequenzen, die eine schnelle Anreicherung eines löslichen Fermentationsproduktes erleichtern (z.B. Antikörper).

$(X_1 ... X_n)$ steht für ein pharmakologisch wirksames Polypeptid oder Protein oder für einen höhermolekularen Vorläufer, aus dem die gewünschte biologisch wirksame Form durch weitere Prozessierung wie Faltung unter Herstellung korrekter Disulfidbrücken und/oder gezielte Spaltung der Polypeptidketten gewonnen wird. Ein Beispiel hierfür wäre Präproinsulin, aus dem das Insulin entsteht.

Die Reste X und Y stehen unabhängig voneinander für natürlich vorkommende Aminosäuren (s. z.B. Schröder, Lübke "The Peptides" Vol. I, New York 1965, Seiten 137-270 und Houben-Weyl "Methoden der organischen Chemie" Bd. 15/1 und 2 (Synthese von Peptiden), Georg Thieme Verlag Stuttgart 1974, Beilage). Insbesondere seien genannt: Gly, Ala, Ser, Thr, Val, Leu, Ile, Asp, Asn, Glu, Gln, Cys, Met, Arg, Lys, Hyl, Orn, Cit, Tyr, Phe, Trp, His, Pro und Hyp.

Unter einer für Oligo- bzw. Polyglycinsequenzen spezifischen Endoprotease wird insbesondere Lysostaphin (Hersteller: [R]SIGMA Chemie GmbH, Deisenhofen; vgl. auch Recsei et al., Proc. Natl. Acad. Sci. USA, Vol. 84, (1987) 1127-1131) verstanden.

Das gewünschte Produkt $(X_1 ... X_n)$ wird mit einem Protein $(Y_1 ... Y_m)$ durch eine beliebige Glycinsequenz (Gly)p + q verbunden. Bevorzugt sind Fusionsproteine, in denen p + q zusammen eine natürliche Zahl zwischen 2 und 30 bedeutet. Eine größere Anzahl aufeinanderfolgender Glycinreste kann dabei aufgrund der besseren Zugänglichkeit der Spaltungsstelle die Geschwindigkeit der Reaktion positiv beeinflussen. Der gleiche Effekt wird aber auch erreicht, wenn kurze Oligoglycinsequenzen von einer oder mehreren anderen Aminosäuren flankiert werden, die das Enzym bei der Spaltung nicht sterisch behindern. Die Länge des Verbindungsstückes ist demnach den strukturellen Eigenschaften der Fusionspartner anzupassen.

Die Spaltungsbedingungen lassen sich in einem sehr weiten Rahmen variieren und damit den Eigenschaften des Fusionsproteins anpassen. So kann das Enzym/Substrat-Verhältnis beispielsweise zwischen 1:1 und 1:1 000 000 liegen und die Reaktion in einem pH-Bereich von 6 bis 9, bevorzugt im Bereich von 7 bis 8, vorzugsweise bei einer Temperatur von 20-60°C, insbesondere von 32-42°C, durchgeführt werden. Gegebenenfalls kann auch je nach dem Grad der Schwerlöslichkeit des Fusionsproteins ein Hilfsstoff, z.B. Harnstoff, Detergentien oder Guanidinhydrochlorid, der das Fusionsprotein in Lösung hält, zugegeben werden. Am schnellsten verläuft die Spaltung zwar, wenn ohne Zusatz von Denaturierungsmitteln eine nahezu vollständige Lösung des Fusionsproteins möglich ist, jedoch wird die Lysostaphin-Endoprotease durch Gegenwart von Harnstoff nicht inaktiviert, sondern die enzymatische Reaktion lediglich verlangsamt. Diese Tatsache kann zur Spaltung besonders schwerlöslicher Fusionsproteine genutzt werden. Andererseits sind prinzipiell bei entsprechender Verlängerung der Reaktionszeit auch Fragmentierungen der lediglich suspendierten Einschlußkörperchen erfolgreich durchführbar. Bei Lösungen des Fusionsproteins - mit oder ohne Denaturierungsmittel - kann die Lysostaphin-Endoprotease auch vorteilhaft in trägergebundener Form (immobilisiertes Enzym) eingesetzt und zur nochmaligen Verwendung wiedergewonnen werden. Als Enzymträger kommen beispielsweise anorganische Träger wie Aluminiumsilikate aber auch polymere, organische Träger wie Agarosen, z.B. [R]Affi-Gel 10 (Fa. Bio Rad), Cellulosen, modifizierte Polyacrylamidgele, die Amino- oder Hydroxylgruppen besitzen oder auch organische Copolymerisate aus Acrylamid, Methacrylaten oder Methacrylamid und Maleinsäureanhydrid in Frage. Die Herstellung entsprechender trägergebundener Enzyme ist beispielsweise beschrieben in Halwachs et al., Biotechnology and Bioengineering XIX (1977) 1667-1677 (Fixierung auf Aluminiumsilikat) oder DE-OS 29 27 534 (Fixierung auf Cellulose).

Das erfindungsgemäße Verfahren eignet sich nicht nur zur selektiven Spaltung von Fusionsproteinen, sondern ist allgemein bei entsprechenden Polypeptiden anwendbar.

Die nachfolgenden Beispiele dienen zur Erläuterung der vorliegenden Erfindung, jedoch ohne daß diese darauf beschränkt wäre. Dabei sollen die Beispiele 3 und 4 belegen, daß die in den Beispielen 1 und 2 erhaltenen Fusionsproteinspaltungen auf den Einbau einer Polyglycinsequenz und nicht auf einen Abbau von $(X_1 ... X_n)$ bzw. $(Y_1 ... Y_m)$ zurückzuführen sind.

**Beispiel 1**

Spaltung eines Polyglycin-haltigen Fusionsproteins

Verwendet wird eine Konstruktion, bei der ein Segment der $\beta$-Galaktosidase über ein $(Gly)_{18}$-Peptid und ein synthetisches Hexapeptid mit Proinsulin verknüpft ist. Proinsulin bildet das Carboxylende des Fusionsproteins. Das Protein ist zu etwa 40 % angereichert.

Dieses Protein wird mit 20 mg/ml Puffer (8 M Harnstoff; 50 mM Tris/HCl; pH 7,5) gelöst und durch langsame Verdünnung mit 50 mM Tris/HCl; pH 7,5 und 8 M Harnstoff pH 7,5 auf verschiedene Harnstoffkonzentrationen (s. Tabelle 1) und eine Proteinkonzentration von 2 mg/ml bzw. 10 mg/ml gebracht. Die jeweils schwach trüben Lösungen werden mit Lysostaphin ([R]SIGMA) im Enzym/Substrat-Verhältnis 1:100 bzw. 1:1000 versetzt und bei 37°C gehalten. Zu bestimmen Zeiten werden Proben entnommen und mittels SDS-Elektrophorese analysiert. Der selektive Abbau des Fusionsproteins an der Polyglycinsequenz ist durch die Abnahme der Fusionsproteinbande und das Entstehen einer neuen Bande für das Galactosidasefragment mit einem um 10000 Dalton niedrigeren Molekulargewicht erkennbar.

Tabelle 1

| Protein-Konzentration (mg/ml) | Enzym/Substrat-Verhältnis | Harnstoff-Konzentration (M) | Reaktionszeit (> 95 % Abnahme der Fusionsprotein-Bande) (Stunden) |
|---|---|---|---|
| 2 | 1 : 100 | 4 | > 20 |
| 2 | 1 : 100 | 3 | 20 |
| 2 | 1 : 100 | 2 | 5 |
| 2 | 1 : 100 | 1 | 2 |
| 10 | 1 : 1000 | 2 | 20 |

In Tabelle 1 sind die Zeiten aufgeführt, nach denen bei densitometrischer Auswertung der SDS-Elektrophorese die Fläche für die ursprünglichen Fusionsbande auf unter 5 % gesunken ist. Die erhaltenen Werte liegen zwischen 1 und 20 Stunden je nach Harnstoffkonzentration und Enzym/Substrat-Verhältnis. Die Anwesenheit eines Reduktionsmittels, z.B. Dithioerythritol (DTE, Cleland's Reagenz), das zur Auflösung des Fusionsproteins vorteilhaft ist, hat auf die Aktivität des Enzyms keinen signifikanten Einfluß.

**Beispiel 2**

Spaltung eines Fusionsproteins in Suspension

Zum Einsatz kommt die gleiche Konstruktion wie in Beispiel 1, allerdings nicht in isolierter, getrockneter Form. Vielmehr wird eine Suspension verwendet, wie sie üblicherweise bei der Gewinnung der Einschlußkörperchen anfällt und die bei ca. 200 g/l Trockensubstanz (90 % Protein) ca. 50 g/l Fusionsprotein enthält. 10 ml dieser Suspension werden mit 50 mM Tris/HCl; pH 7,5 auf 200 ml verdünnt und mit 20 mg Lysostaphin ((R)SIGMA) versetzt. Nach 20 Stunden bei 37°C zeigt die SDS-Gelelektrophorese nur noch geringe Spuren nicht fragmentierten Fusionsproteins.

**Beispiel 3**

Inkubation von Proinsulin mit Lysostaphin

Proinsulin (Human) wird mit 1 mg/ml in 50 mM Tris/HCl; pH 7,5 gelöst und mit Lysostaphin im Enzym/Substrat-Verhältnis 1:100 versetzt. Die Lösung wird bei 37°C gehalten. Nach 1, 3, 5 und 20 Stunden werden Proben entnommen und mittels Reversed Phase-HPLC analysiert. Dabei ist kein Hinweis auf einen Abbau des Proinsulins durch Lysostaphin zu erkennen.

**Beispiel 4**

Versuch zur Spaltung eines Fusionsproteins ohne Polyglycinsequenz

Verwendet wird eine Konstruktion, bei der ein Segment der $\beta$-Galaktosidase über ein synthetisches Hexapeptid mit Proinsulin verknüpft ist. Proinsulin bildet das Carboxylende des Fusionsproteins. Das Fusionsprotein ist zu etwa 80 % angereichert. Dieses Protein wird mit 20 mg/ml Puffer (8 M Harnstoff; 50 mM Tris/HCl; pH 7,5) gelöst und durch langsame Verdünnung mit 50 mM Tris/HCl; pH 7,5 auf eine Proteinkonzentration von 2 mg/ml gebracht. Die schwach trübe Lösung wird mit Lysostaphin ((R)SIGMA) im Enzym/ Substrat-Verhältnis 1:100 versetzt und bei 37°C gehalten. Die Analyse von Proben durch SDS-Elektrophorese zeigt selbst nach 20 Stunden keinen Abbau des Fusionsproteins.

**Patentansprüche**

**1.** Verfahren zur Herstellung eines Polypeptids oder Proteins durch enzymatische Spaltung, dadurch gekennzeichnet, daß ein Fusionsprotein mit der Sequenz

$(Y_1 .... Y_m)$ - $(Gly)_{p+q}$-$(X_1...X_n)$

wobei $(Y_1...Y_m)$-$(Gly)_p$ die abzuspaltende Sequenz und $(Gly)_q$-$(X_1...X_n)$ das Polypeptid oder Protein darstellen,

X und Y      unabhängig voneinander natürliche Aminosäuren,

m und n      Zahlen größer als 1,

p bzw. q      eine Zahl größer als 0 und

p + q      zusammen eine natürliche Zahl zwischen 2 und 100 bedeuten, falls Y für Gly steht, kann p + q auch < 2 und p = 0 sein und falls $X_1$ für Gly steht, kann p + q auch < 2 und q = 0 sein,

mit einer für Oligo- bzw. Polyglycinsequenzen spezifischen Endoprotease gespalten wird und das dabei freigesetzte Polypeptid oder Protein gegebenenfalls einer weiteren chemischen oder enzymatischen Behandlung unterzogen oder direkt weiterverarbeitet wird.

**2.** Verfahren gemäß Anspruch 1, dadurch gekennzeichnet, daß als Endoprotease Lysostaphin eingesetzt wird.

**3.** Verfahren gemäß Anspruch 1, dadurch gekennzeichnet, daß p + q zusammen eine ganze Zahl zwischen 2 und 30 bedeuten.

**4.** Verwendung eines Fusionsproteins gemäß Anspruch 1 zur Herstellung eines pharmakologisch wirksamen Polypeptides.

**Claims**

**1.** A method for the preparation of a polypeptide or protein by enzymatic cleavage, which comprises cleavage of a fusion protein having the sequence

$(Y_1 ... Y_m) - (Gly)_{p+q}-(X_1...X_n)$

where $(Y_1...Y_m)$-$(Gly)_p$ represents the sequence which is to be eliminated and $(Gly)_q$-$(X_1...X_n)$ represents the polypeptide or protein,

X and Y      denote, independently of one another, natural amino acids,

m and n      denote numbers greater than 1,

p and q      each denotes a number greater than 0 and

p + q      together denote a natural number between 2 and 100, and if $Y_m$ represents Gly it is possible for p + q also to be < 2 and p to be 0, and if $X_1$ represents Gly it is possible for p + q also to be < 2 and q to be 0,

with an endoprotease specific for oligo- or polyglycine sequences, and subjecting the polypeptide or protein which is liberated by this to further chemical or enzymatic treatment where appropriate, or directly processing it further.

**2.** The method as claimed in claim 1, wherein lysostaphin is used as endoprotease.

**3.** The method as claimed in claim 1, wherein p + q together denote a whole number between 2 and 30.

**4.** The use of a fusion protein as claimed in claim 1 for the preparation of a pharmacologically active polypeptide.

**Revendications**

**1.** Procédé de préparation d'un polypeptide ou d'une protéine par clivage enzymatique, caractérisé en ce qu'on coupe, à l'aide d'un endoprotéase spécifique pour les séquences oligo- et polyglycines, une protéine de fusion de séquence

$(Y_1...Y_m) - (Gly)_{p+q} - (X_1...X_n)$

dans laquelle $(Y_1...Y_m)$ - $(Gly)_p$ représente la séquence à cliver et $(Gly)_q$ - $(X_1...X_n)$ représente le polypeptide ou la protéine,

X et Y, indépendamment l'un de l'autre, sont des acides aminés naturels,

m et n sont des nombres supérieurs à 1,

p ou q est un nombre supérieur à 0, et

p + q ensemble représentent un nombre naturel valant de 2 à 100, et, au cas où $Y_m$ est Gly, p + q peut aussi être < 2 et p être 0, et, au cas où $X_1$ est Gly, p + q peut aussi être < 2 et p être 0,

et en ce qu'on soumet éventuellement le polypeptide ou la protéine ainsi libéré à un autre traitement chimique ou enzymatique, ou en ce qu'on poursuit directement sa transformation.

**2.** Procédé selon la revendication 1, caractérisé en ce qu'on utilise de la lysostaphine, comme endoprotéase.

**3.** Procédé selon la revendication 1, caractérisé en ce que p + q ensemble représentent un nombre entier compris entre 2 et 30.

4. Utilisation d'une protéine de fusion de la revendication 1 pour la préparation d'un polypeptide pharmacologiquement actif.